Europäisches Patentamt

European Patent Office

Office européen des brevets

(11) Publication number: **0 240 105 B1**

# EUROPEAN PATENT SPECIFICATION

(45) Date of publication of patent specification: **21.07.93** (51) Int. Cl.5: **C12N 15/52**, C12P 17/18

(21) Application number: **87300716.5**

(22) Date of filing: **28.01.87**

(54) A gene coding for biotin synthetase and utilization thereof.

(30) Priority: **25.03.86 JP 66532/86**
**24.04.86 JP 95724/86**
**12.01.87 JP 4404/87**

(43) Date of publication of application:
**07.10.87 Bulletin 87/41**

(45) Publication of the grant of the patent:
**21.07.93 Bulletin 93/29**

(84) Designated Contracting States:
**CH DE FR GB LI**

(56) References cited:
**WO-A-87/01391**

**CHEMICAL ABSTRACTS, vol. 90, no. 9, 26th February 1979, page 250, abstract no. 69004e, Columbus, Ohio, US; G. COHEN et al.: "Isolation and characterization of a Co1E1 plasmid containing the entire bio gene cluster of Escherichia coli K12", & MOL. GEN. GENET. 1978, 166(3), 305-12**

(73) Proprietor: **NIPPON ZEON CO., LTD.**
**6-1, 2-chome, Marunouchi, Chiyoda-ku Tokyo(JP)**

(72) Inventor: **Ohsawa, Ikurou**
**11-11, Nakaochiai-3-chome Shinjuku-ku Tokyo(JP)**
Inventor: **Fujii, Nobuko**
**26-27, Yokodai-3-chome Isogo-ku Yokohama(JP)**
Inventor: **Kamogawa, Kouichi**
**2153-42, Kamariyacho Kanazawa-ku Yokohama(JP)**
Inventor: **Lemoine, Yves**
**4, rue des Alisiers F-67100 Strasbourg Neudorf(FR)**
Inventor: **Gloeckler, Remi**
**22, rue de Stockholm F-67000 Strasbourg(FR)**
Inventor: **Speck, Denis**
**4, rue du Bitzen F-67200 Eckbolsheim(FR)**
Inventor: **Kisou, Tsugio**
**17-13, Shinoharanishimachi Kohoku-ku Yokohama(JP)**
Inventor: **Hayakawa, Kazuko**
**14-7, Senda Koto-ku Tokyo(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACT, vol. 93, no. 7, 18th August 1980, page 480, abstract no. 65744k, Columbus, Ohio, US; A. MUKHERJEE et al.: "Construction and characterization of a recombinant plasmid containing the entire biotin genes of E. coli K12", & PLASMIDS TRANSPOSONS, [PROC. ANNU. SYMP. SCI. BASIS MED.], 4th 1979 (Pub. 1980), 379-86

J. MOL. BIOL., vol. 148, 1981, pages 63-76, Academic Press Inc. Ltd, London, GB; A. SANCAR et al.: "Identification of the uvrB Gene Product"

GENE, vol. 1, 1977, pages 331-345, Elsevier/North-Holland Biomedical Press, Amsterdam, NL; C.K. DAS GUPTA et al.: "Isolation and characterization of the biotin genes of Escherichia coli K-12"

AGRI. BIOL. CHEM., vol. 47, no. 5, 1983, pages 1011-1016; H. YAMADA et al.: "Biotin overproduction by biotin analog-resistant mutants of bacillus sphaericus"

CHEMICAL ABSTRACTS, vol. 106, no. 3, 19th January 1987, page 209, abstract no. 14236a, Columbus, Ohio, US

⑦④ Representative: **Harrison, David Christopher et al**
**MEWBURN ELLIS 2 Cursitor Street**
**London EC4A 1BO (GB)**

**Description**

This invention relates to a deoxyribonucleic acid (DNA) coding for genetic information which participates in the biosynthetic reaction of biotin from desthiobiotin and utilization thereof. More particularly, it relates to the above DNA, a recombinant vector to which the above DNA has been inserted, bacteria containing said recombinant vector, and a process for producing biotin by use thereof.

Biotin is an essential vitamin for animals, plants, microorganisms, etc. and its industrial production using a genetic recombination technique is eagerly desired. The analysis of genetic information involved in the biosynthetic reaction in the biosynthetic reaction of biotin has been advanced in recent years on Escherichia coli (Journal of Bacteriology 94, 2065 (1967), ibid. 112, 830 (1972), Nature 276, 689 (1978), etc.), and a biotin production technique using E. coli is investigated by application of a genetic recombination technique using the analysis results. However, such a method has the following defect. For example, when desthiobiotin is used as a biotin precursor (Japanese Patent Application Kokai (Laid-Open) No. '86-149091), the amount of biotin produced is about 12 $\mu$g/ml, and when glucose is used as a carbon source without using any precursor (Japanese Patent Application Kokai (Laid-Open) No. '86-202686), the amount of biotin produced is about 2 $\mu$g/ml. Thus, the amount of biotin produced is small in both cases.

On the other hand, there is also known a method for producing biotin by culturing a microorganism belonging to the genus Bacillus, and there have been found bacteria which produce a much larger amount of biotin as compared with E. coli (for example, Japanese Patent Application Kokai (Laid-Open) Nos. '81-160998, '83-60996 and '83-152495, Yamada et al., Agric. Biol. Chem. 47(5) 1011-1016, (1983)). However, there has been made substantially no analysis of genetic information involved in the biosynthesis of biotin by bacteria of the genus Bacillus, and therefore it has been very difficult to apply the genetic information to a genetic recombination technique.

In this state of the prior art the present inventors studied in order to obtain DNA coding for genetic information involved in the biosynthesis of biotin from desthiobiotin which is the final step of the biosynthesis of biotin from bacteria of the genus Bacillus, and consequently succeeded in obtaining DNA coding for genetic information involved in the biosynthetic reaction of biotin (hereinafter sometimes referred to as DNA coding for biotin synthetase). Moreover, the present inventors found that bacteria transformed with a recombinant vector to which the above DNA had been inserted had a high biotin productivity, whereby this invention has been accomplished.

The first aspect of this invention is to provide DNA coding for genetic information involved in the biosynthetic reaction of biotin from desthiobiotin derived from bacteria of the genus Bacillus. The second aspect of this invention is to provide a recombinant vector to which the above DNA alone or both the above DNA and a promoter have been inserted. The third aspect of this invention is to provide bacteria transformed or transduced with said recombinant vector. The fourth aspect of this invention is to provide a process for producing biotin by culturing said bacteria.

BRIEF DESCRIPTION OF THE DRAWINGS

Figs. 1 to 3 show the restriction endonuclease cleavage maps of pSB01 plasmid DNA, pSB03 plasmid DNA, and pSB16 plasmid DNA, respectively.

Figs. 4 and 5 show the restriction endonuclease cleavage map and the base sequence and amino acid sequence, respectively, of DNA fragment from a bacterium of the genus Bacillus inserted into pSB16.

Figs. 6 and 8 show methods for preparing pSB301 plasmid DNA and pBHB13 plasmid DNA, respectively.

Fig. 7 shows the base sequence of synthetic DNAs Bbgl-1 and Bbgl-2.

Fig. 9 shows the sequence at the junction of $\alpha$-amylase promoter region to the biotin synthetase gene.

DESCRIPTION OF THE PREFERRED EMBODIMENTS

As the microorganism used for obtaining DNA coding for biotin synthetase in this invention, any one may be selected from the group consisting of strains newly isolated from the nature, known cultured strains, and strains obtained therefrom by treatment according to a conventional mutation inducing method (e.g., a method comprising physical treatment using ultraviolet ray, X-ray or $\gamma$-ray irradiation, etc., or chemical treatment using agents such as nitrosoguanidine, etc.), so long as it is one of biotin-producing strains belonging to the genus Bacillus, especially, Bacillus sphaericus, that is, those containing the enzymatic systems for biosynthesis of biotin. Furthermore, there may also be used strains obtained from the above-mentioned strains by imparting thereto resistance to either or both of actithiazic acid (i.e., Acidomycin) and 5-(2-thienyl)valeric acid in order to increase the biotin productivity. Specific examples of such strains include, for example, Bacillus sphaericus IFO 3525 and its mutant strains obtained by treatment with N-methyl-N′-nitro-N-nitrosoguanidine (FERM P-6422, FERM BP-1098 and FERM P-6143).

A method for separating DNA coding for biotin synthetase from these bacterial strains is not critical. For example, it is sufficient that there is employed a method comprising isolating and purifying total DNAs from the bacterial strains described above by a conventional method, cleaving the same with restriction enzymes, ligating cleaved DNAs enzymatically with a vector cleaved by restriction enzymes which can produce in DNA the same cohesive end as in the case of the above restriction enzymes, transforming (or transducing) a biotin auxotrophic mutant strain which lacks the biosynthetic ability to produce biotin from desthiobiotin by use of the recombinant vector thus obtained, and selecting the bacterial strains having biotin productivity from the transformants thus obtained.

Needless to say, the DNAs used in the above include modified DNAs having substantially the same function as that of the DNAs, namely, those in which a part of the base sequence is substituted, inserted or deleted but which have the same function of that of the DNAs.

As the above DNAs, there are preferably used those containing Shine-Dalgarno sequence, a translation initiation codon and a translation termination codon.

In constructing the recombinant vector, plasmid vectors, λgt type vectors, etc. are used, and the biotin productivity of the transformed or transduced bacteria can be improved by, if necessary, inserting a promoter.

In this invention, as a vector for constructing the recombinant vector, any vector may be used so long as it can transform (or transduce) the above-mentioned biotin auxotrophic mutants. For example, when a biotin auxotrophic mutant of Escherichia coli is used for the transformation (or transduction), there can be used EK system plasmid vectors such as pSC101, pRK353, pRK646, pRK248, pDF41, etc. of stringent type, and ColE1, pVH51, pAC105, RSF2124, pCR1, pMB9, pBR313, pBR322, pBR324, pBR325, pBR327, pBR328, pKY2289, pKY2700, pKN80, pKC7, pKB158, pMK2004, pACYC1, pACYC184, pUC8, pUC9, etc. of relax type; and λgt system phage vectors such as λgt•λC, λgt•λB, λWES•λC, λWES•λB, λZJvir•λB′, λALO•λB, λWES•T$_5$622, λDam, etc. Among these vectors, the plasmid vectors are particularly preferred.

The DNA coding for biotin synthetase which is contained in the present recombinant vector should be inserted under the control of and downstream to DNA having a promoter sequence which functions in the above-mentioned transformant (the term "promoter" used in the present specification and Claims means DNA having a promoter sequence).

As the promoter usable in this invention, either natural one or synthetic one may be used so long as a recombinant vector having the promoter inserted therein can function in host bacteria. As such a promoter, it is considered to be possible to use lac promoter, lpp promoter, P$_L$ promoter, P$_R$ promoter, trp promoter, Tac promoter, etc. all functionable in Escherichia coli, in the case where bacteria of the genus Escherichia are used as a host; α-amylase promoter derived from bacteria of the genus Bacillus (see, for example, J. Biochem. 98, 1147 (1985), J. Bacteriol. 158, 369 (1984), and Gene 23, 267 (1983)), penicillinase promoter derived from Bacillus licheniformis, trp promoter derived from Bacillus subtilis, 0.3 Kb promoter of phage SP02, etc. in the case where bacteria of the genus Bacillus are used as host; and catechol-2,3-oxygenase promoter derived from bacteria of the genus Pseudomonas, in the case where bacteria of the genus Pseudomonas are used as a host. As a natural promoter, one which is contained in a vector used in the form of a recombinant vector may be used as it is.

As the above-mentioned biotin auxotrophic mutant strain, any strain may be used so long as the expression of the DNA coding for genetic information derived from a bacterium of the genus Bacillus is possible in the cells of said strain, and such a mutant can be obtained with good reproducibility by a well-known method (for example, J. Bacteriol. 115, 662 (1973), Proc. Natl. Acad. Sci. in USA 69, 2219 (1972), and Virology 42, 474 (1970)).

There can be used, for example, a bacterial strain having no biosynthetic ability to produce biotin from desthiobiotin (hereinafter referred to as Escherichia coli K3) which is obtained by subjecting Escherichia coli C600 (Proc. Natl. Acad. Sci. in USA 71, 4579 (1974)) to mutagenic treatment according to the method described in J. Bacteriol. 94, 1930 (1967).

A method for the transformation is also not critical. For example, when the above Escherichia coli K3 is used as biotin auxotrophic mutant, the transformation can be conducted according to a conventional method which comprises treating cells with calcium chloride (see, for example, Journal of Molecular Biology 53, 154 (1970)).

When bacterial strains having biotin productivity newly imparted by a recombinant vector having, as an insert, DNA coding for biotin synthetase derived from a bacterium of the genus Bacillus is selected from the thus obtained transformants of biotin auxotrophic mutant strains, it is sufficient that there is employed, for example, a method comprising subjecting said transformants to screening by use of media having the same composition as that of media in which the parent biotin auxotrophic mutant strain can grow, except that biotin is substantially omitted, and then selecting bacterial strains which can grow in the biotin-deficient media.

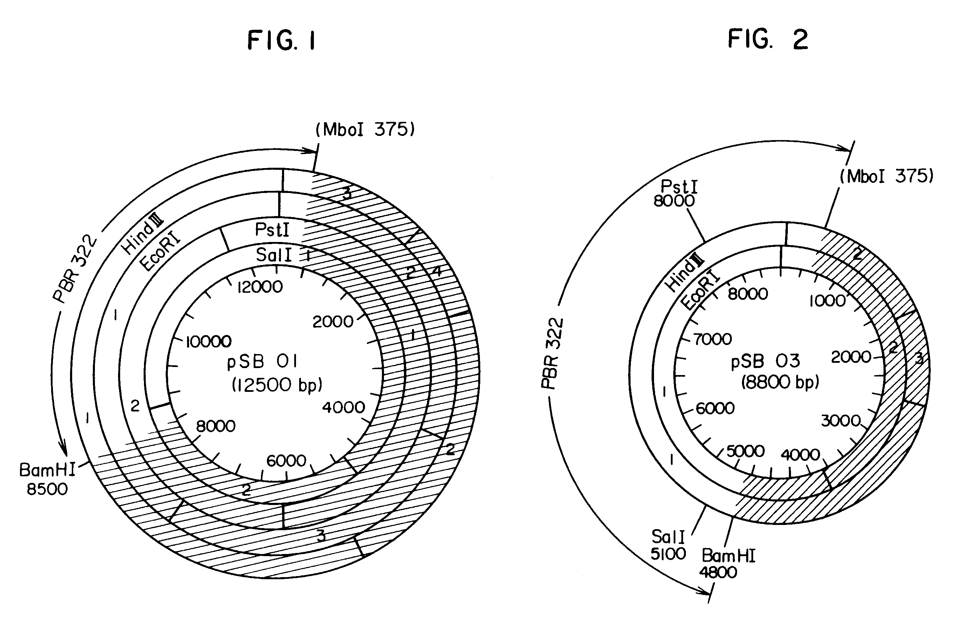

One of the transformed bacterial strains thus obtained was named Escherichia coli K3 (pSB01) and a hybrid plasmid from cultured cells of Escherichia coli K3 (pSB01) was named pSB01. This pSB01 corresponds, as shown in Example 1 hereinafter, to plasmid pBR322 having a DNA fragment from Bacillus sphaericus inserted therein, and its restriction endonuclease cleavage map is shown in Fig. 1. From Fig. 1, it can be understood that the DNA coding for genetic information involved in the biosynthesis of biotin which is contained in pSB01 is present in the DNA fragment of 8.2 kb in length which starts from Mbo I site, followed by Hind III, Hind III, EcoR I, Sal I, Hind III, Pst I, EcoR I, and BamH I sites in this order (the shaded part in Fig. 1). The Mbo I site in this map shows the starting point, but Fig. 1 does not eliminate the possibility of presence of other Mbo I sites in the DNA.

Needless to say, the recombinant vector having the DNA coding for biotin synthetase of this invention inserted therein is not limited to pSB01. For example, it is also possible to newly induce a plasmid having the above DNA inserted therein from pSB01 by utilizing the intrinsic restriction enzyme cleavage sites of pSB01. Concretely, pSB01 is completely digested, for example, with restriction enzyme EcoR I, and then enzymic re-ligation reaction is carried out, after which a bacterial strain having no biosynthetic ability to produce biotin from desthiobiotin is transformed using the plasmid mixture thus obtained, and a bacterial strain having biotin productivity and a smaller plasmid than that of pSB01 is selected from the transformed strains thus obtained.

One of the transformed bacterial strains obtained in the manner described above was named Escherichia coli K3 (pSB03), and a hydrid plasmid isolated from the cultured cells of Escherichia coli K3 was named pSB03. The restriction endonuclease cleavage map of pSB03 is as shown in Fig. 2. It can be understood from Fig. 2 that the DNA coding for biotin synthetase is present in the DNA fragment of about 4.5 kb in length which starts from Mbo I site, followed by Hind III, Hind III, EcoR I and BamH I sites in this order (the shaded part in Fig. 2). This approx. 4.5 kb DNA fragment is a fragment formed by the ligation of the approx. 3.5 kb portion (i.e., the portion from Mbo I site to EcoR I site) and the approx. 3 kb portion (i.e., the portion from EcoR I site to BamH I site) in the approx. 8.2 kb parent DNA fragment. When the approx. 1 kb DNA fragment was separately inserted into pBR322 and a biotin auxotrophic mutant was transformed with the thus formed recombinant vector, the transformant thus obtained had no biotin productivity. From these facts, it was confirmed that the portion coding for biotin synthetase was present in the approx. 3.5 kb fragment between Mbo I and EcoRI sites.

A bacterial strain having a still smaller plasmid can be obtained similarly by digesting pSB03, for example, with restriction enzyme Hind III, ligating the resulting digest to another vector which has also been cleaved using Hind III, transforming a bacterial strain having no biosynthetic ability to produce biotin from desthiobiotin with the thus obtained plasmid mixture, and selecting a bacterial strain having biotin productivity from the transformed bacterial strains thus obtained.

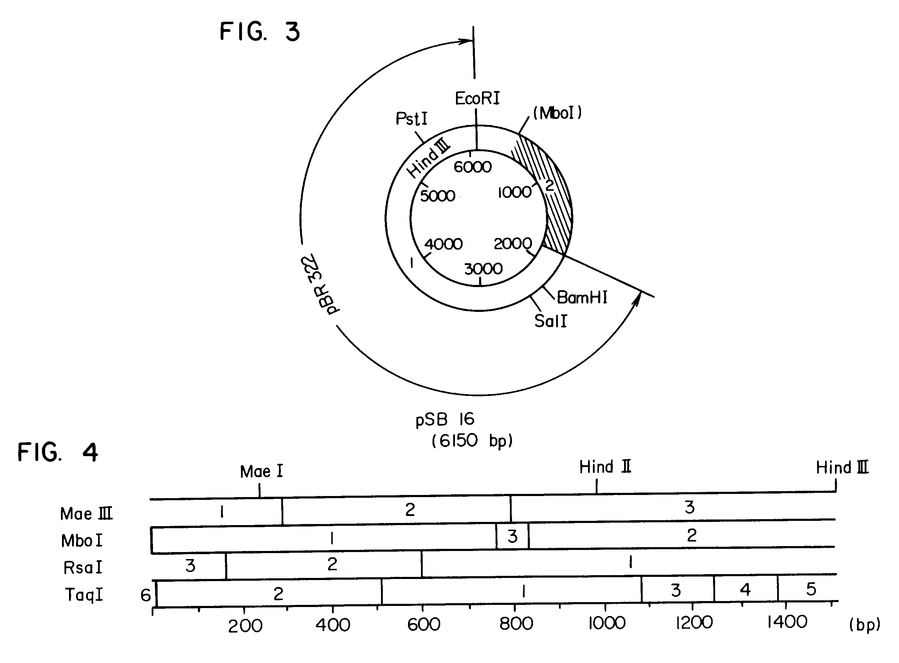

One example of transformed strain obtained in the manner described above is Escherichia coli K3 (pSB16). The restriction endonuclease cleavage map of plasmid pSB16 present in this transformed bacterial strain is as shown in Fig. 3, and the restriction endonuclease cleavage map of the DNA fragment from a bacterium of the genus Bacillus inserted therein is as shown in Fig. 4. From these results, it can be understood that the DNA coding for biotin synthetase is present in the approx. 1.5 kb DNA fragment which starts from Mbo I site, followed by Taq I, Rsa I, Mae I, Mae III, Taq I, Rsa I, Mbo I, Mae III, Mbo I, Hind II, Taq I, Taq I, Taq I, and Hind III sites in this order.

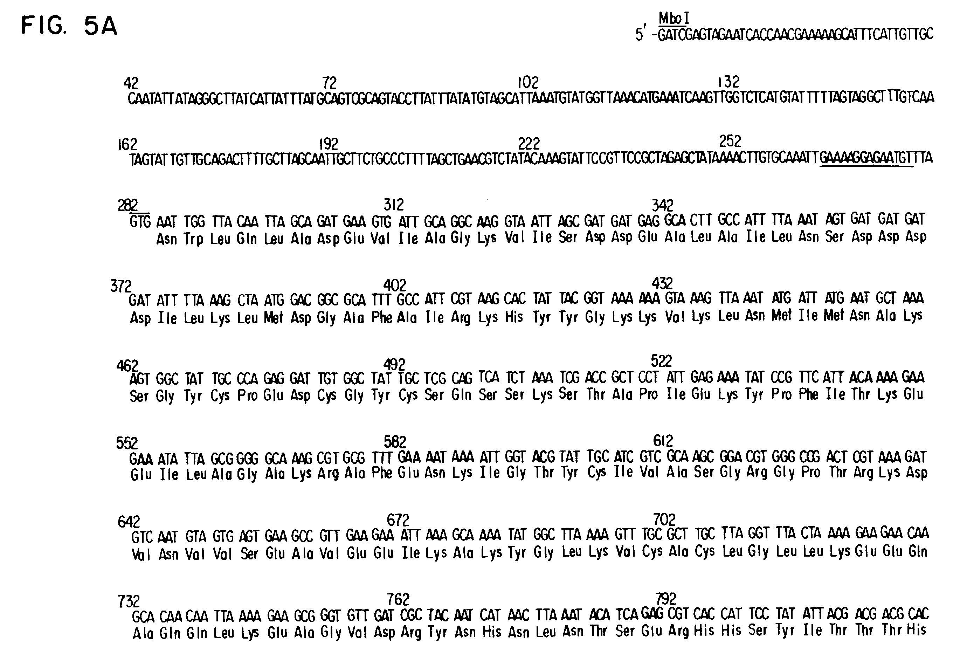

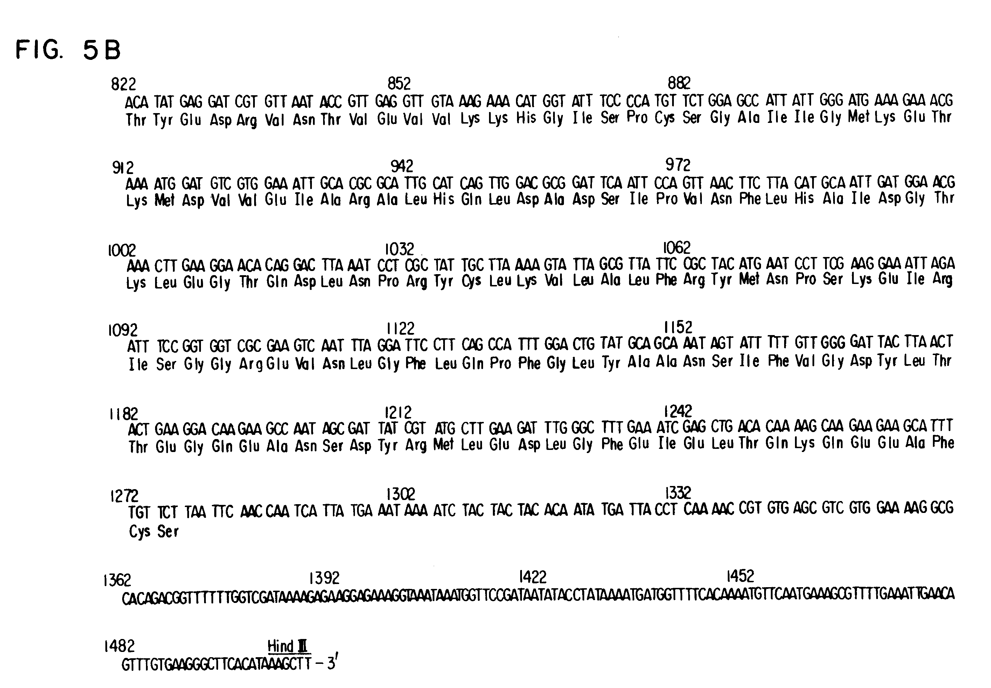

The sequence of this approx. 1.5 kb DNA fragment was analyzed according to the method described in Example 3 hereinafter to find that its base sequence was as shown in Fig. 5. This sequence has one large open reading frame from the 5′-terminal on the MboI side toward the 3′-terminal on the Hind III side. The GTG sequence which is one of translation initiation codons of procaryotes (Ann. Rev. Microbiol. 35, 365 (1981)) is present in the region from the 5′-terminal to the positions 282-284, and 332 codons are linked to GTG as far as the positions 1278-1280 at which the translation termination codon TAA is present. Shine-Dalgarno sequence is present at the positions 265-278 upstream to this GTG.

The amino acid sequence coded for by said DNA fragment can be deduced as shown in Fig. 5 from its base sequence. The polypeptide having such an amino acid sequence is composed of 331 amino acids, and its molecular weight is calculated to be 36,888 dalton. In this case, it is conceivable that methionine coded for by the translation initiation codon GTG remains, so that the polypeptide is composed of 332 amino acids. The polypeptide functions as an enzyme which catalyzes the biosynthesis of biotin from desthiobiotin in bacteria.

In the case of bacteria of the genus Bacillus, it is known that some enzymes having the same function are different from one another in amino acid sequence depending on the strain (J. Bacteriol. 158, 369 (1984)). In such enzymes, a part of the amino acids are substituted, deleted or inserted and in some cases, the amino acid sequence itself is not changed at all even if the base sequence is changed. Therefore, in some cases, the base sequence of DNA coding for biotin synthetase obtained from a strain other than Bacillus sphaericus and the amino acid sequence deducible from this base sequence are somewhat different from the base sequence and the amino acid sequence shown in Fig. 5. Needless to say, the amino acid sequence and the base sequence of this DNA fragment are artificially changed in some cases. Concretely, there is, for example, the case where as shown in Example 6 hereinafter, initiation sequence GTG is converted into initiation sequence ATG. In either case, this invention includes polypeptides coded for by DNA fragments which polypeptides have been modified in such a range that they have a substantially unchanged function as biotin synthetase, namely, polypeptides in which a part of the amino acid sequence or the base sequence is substituted, inserted or deleted. Although the limit of the change are not always invariable, any polypeptide of which DNA sequence is hydridizable with the base sequence shown in Fig. 5 is expected to function as the biotin synthetase.

As the host of recombinant vector used in this invention, there can be used, for example, bacteria belonging to the genus Escherichia such as Escherichia coli, etc.; bacteria belonging to the genus Bacillus such as Bacillus subtilis, Bacillus sphaericus, etc.; and bacteria belonging to the genus Pseudomonas such as Pseudomonas putida, Pseudomonas aeruginosa, etc.

In order to obtain a transformant excellent in biotin productivity by transforming (or transducing) these hosts, as mentioned previously, the DNA coding for genetic information involved in the biosynthetic reaction of biotin from desthiobiotin should be replicable in the host bacteria and should be inserted in the form in which it is under the control of a promoter which can function in the host bacteria. The promoter is inserted upstream to the above DNA and is preferably located in the vicinity of the DNA. Concretely, it is inserted at a distance therefrom of 300 bases or less, more preferably 150 bases or less.

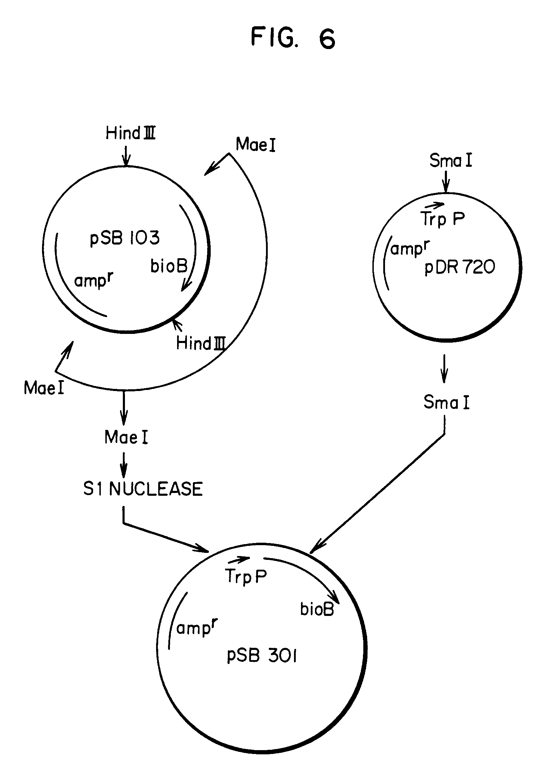

The recombinant plasmid vector containing said DNA under the control of the promoter is prepared by the following procedure. For example, a region including said DNA is excised from a plasmid containing said DNA such as pSB01, pSB03, pSB16, pSB103, etc. with restriction enzymes, and for example, when trp promoter is used, the DNA obtained by the excision is inserted downstream to the promoter sequence of pDR720 (Gene 20, 231 (1982)). As the host bacteria belonging to the genus Escherichia, there can be used bacteria often used in the field of genetic engineering such as Escherichia coli strains C 600, JM 103, MC 169, CAG 1138, JE 8476, GC 4670, AB 1899, JE 8475, GW 5229, etc., strains newly isolated from the nature, known cultured strains, and strains derived therefrom by the conventional mutation inducing methods, though Escherichia coli strains having a low intracellular protease activity such as MC 169, etc. are particularly preferred. One example of recombinant vector thus obtained is pSB301 shown in Fig. 6. It can be understood that the DNA conding for biotin synthetase is inserted under the control of trp promoter, in the vicinity thereof.

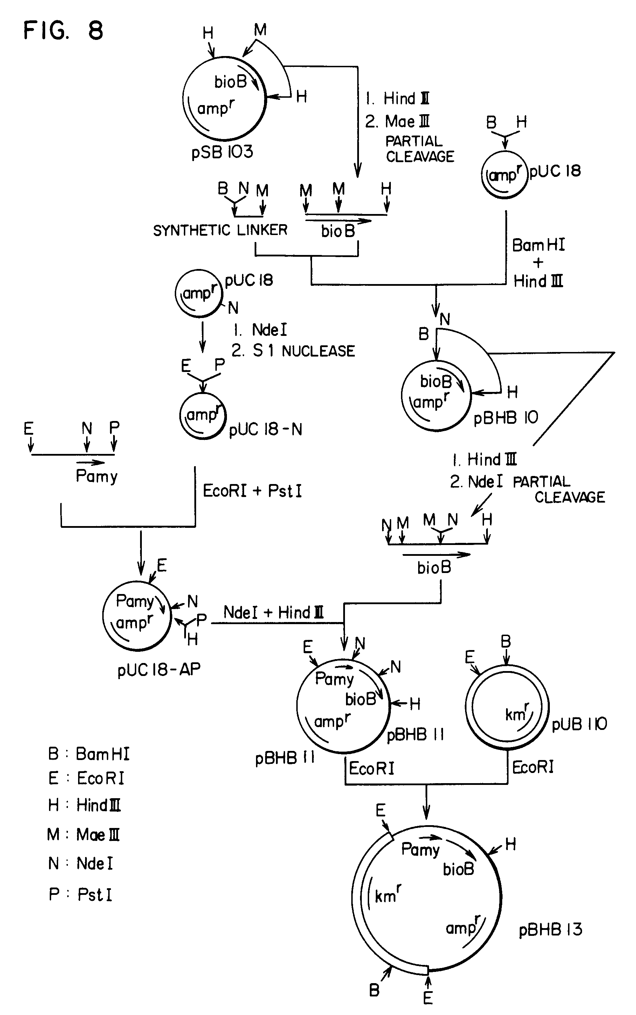

When a microorganism belonging to the genus Bacillus is used as a host, there can be used as a promoter, as described above, for example α-amylase promoters of Bacillus licheniformis and Bacillus stearothermophilus which are thought to function generally in bacteria belonging to the genus Bacillus. When the DNA is linked to these promoters, a synthetic linker or the like may be used as shown in Example 6, in order that the 5′-side sequence containing translation initiation codon GTG sequence of the DNA fragment shown in Fig. 5 is the same as the 3′-side sequence of the promoter. Needless to say, when a microorganism belonging to the genus Bacillus is used as a host, the recombinant vector completed should be replicable in the host, and for this purpose, a part of said vector should contain a replication origin of a plasmid which is thought to be replicable generally in bacteria belonging to the genus Bacillus, such as pUB110. As to the host bacteria belonging to the genus Bacillus, bacteria classified into Bacillus subtilis often used in the field of genetic engineering, biotin-producing bacteria belonging to Bacillus sphaericus usable for colleting genetic information involved in the biosynthetic reaction of biotin, for instance, DNA coding for biotin synthetase, etc., are all usable for the objects of this invention. One example of recombinant vector thus obtained is pBHB13 shown in Fig. 8. It can be understood that this vector has the DNA involved in biotin synthetase inserted under the control of α-amylase promoter derived from Bacillus licheniformis and contains the replication origin of pUB110.

Similarly, when a microorganism belonging to the genus Pseudomonas is used as a host, catechol-2,3-oxygenase promoter or the like is used as a promoter and the replication origin of plasmid RSF 1010 or its derivative (In Plasmids of Medical, Environmental and Commercial Importance, p. 411 (1979)) is incorporated into a part of a recombinant vector to be prepared.

In this invention, by use of the recombinant vectors obtained in the manner described above, the suitably chosen host bacteria are transformed to obtain transformants. As transformation methods employed in this case, there may be employed conventional methods suitable for bacteria of the genus Escherichia, bacteria of the genus Bacillus and bacteria of the genus Pseudomonas, respectively. For example, the transformation of Bacillus sphaericus, a microorganism of the genus Bacillus, can be carried out according to the process described in Journal of General Microbiology, 130, 203 (1984).

This invention provides also a process for producing biotin which comprises culturing the thus obtained transformant to allow the same to produce biotin, and separating and purifying the biotin produced. In the production of biotin, a biotin precursor added to a culture medium used for the culture varies depending on whether or not the host cells to be transformed by use of the above-mentioned recombinant vector has desthiobiotin productivity. For example, when biotin is produced by culturing a transformant obtained by transforming a microorganism of the genus Escherichia having substantially no desthiobiotin producvvity with pSB301, desthiobiotin should be added to the culture medium, as described in Example 7. On the other hand, when a microorganism capable of producing desthiobiotin by utilizing desthiobiotin precursors such as pimelic acid and azelaic acid, for example, Bacillus sphaericus IFO 3525 or Bacillus sphaericus NCIB 9370 is used as a host, it is not always necessary to add desthiobiotin, and mere addition of pimelic acid or azelaic acid as a desthiobiotin precursor to the culture medium is sufficient. The adding amount of desthiobiotin or its precursor is not critical and may properly be selected depending on the biotin productivity of the host cells.

As the culture medium used for culturing the microorganism of this invention, there can be used either synthetic culture media containing carbon sources, nitrogen sources and inorganic substances, or natural media. As the carbon sources, there can be used carbohydrates such as glucose, glycerol, fructose, sucrose, maltose, mannose, starch, starch hydrolyzate, molasses, etc. As the nitrogen sources, there can be used various inorganic and organic ammonium salts such as ammonia, ammonium chloride, ammonium phosphate, ammonium sulfate, ammonium carbonate, ammonium acetate, etc., and natural organic nitrogen sources such as meat extract, yeast extract, corn steep liquor, casein hydrolyzate, defatted soy bean cake, digests thereof, etc. Many of the natural organic nitrogen sources can serve both as nitrogen sources and carbon sources. As the inorganic substances, there can be used potassium dihydrogen-phosphate, dipotassium hydrogen phosphate, magnesium sulfate, sodium chloride, ferrous sulfate, calcium chloride, zinc chloride, copper sulfate, manganese chloride, cobalt chloride, ammonium molybdate, sulfuric acid, etc.

When the microorganism prepared has newly acquired the resistance to antibacterial agents, the addition of the corresponding antibacterial agent to a culture medium permits both the prevention of falling-off of a plasmid DNA having antibacterial agent resistance as a marker which the microorganism possesses and the prevention of contamination by contaminants.

In addition, when some or all of the reactions are inhibited by biotin in a culture medium in the course of biosynthesis of biotin, the addition of a desthiobiotin precursor to the biotin-free medium is conducted preferably at the last stage of logarithmic growth phase of cells (see USP No. 4,563,624).

The cultivation is conducted preferably under aerobic conditions, for example, shaking culture or cultivation with stirring and aeration and is carried out usually at 20 - 45°C for about 1 to 5 days. In this case, it is necessary to satisfy conditions where a promoter which transcribes biotin synthetase gene functions properly. For example, when trp promoter is used in bacteria of the genus Escherichia, the transcriptional activity of the promoter can be induced by adding indoleacrylic acid.

EFFECTS OF THE INVENTION

Thus, according to this invention, there is provided a DNA fragment coding for biotin synthetase derived from a microorganism of the genus Bacillus having biotin productivity; bacteria excellent in biotin productivity can be obtained by transforming (or transducing) bacteria with a recombinant vector having said DNA fragment inserted therein; and there is provided a process for producing biotin efficiently by culturing these bacteria.

Examples

This invention is more concretely explained with reference to the following examples.

Example 1

(1) Preparation of total DNA fragments

The resistant strain (FERM BP-1098) of Bacillus sphaericus IFO 3525 to both actithiazic acid and 5-(2-thienyl)-valeric acid was incubated with shaking in 1 liter of LB culture media (1% bactotryptone, 0.5% yeast extract, 1% NaCl, 0.1% glucose; pH 7.5) at 28°C for about 5 hours, and bacterial cells were harvested at the logarithmic growth phase by centrifugation. Total DNAs were extracted from the bacterials cells by the Phenol method (Biochem. Biophys. Acta, 72, 619 - (1973)), and purified to obtain finally 3.0 mg of total DNAs.

Restriction endonuclease Mbo I was added to 500 $\mu$g of the total DNAs obtained, and the resulting mixture was subjected to reaction at 37°C for 30 minutes to cleave the DNAs partially, after which the reaction mixture was subjected to agarose gel electrophoresis and DNA fragments of about 4 to about 10 kb were recovered.

(2) Cleavage of vector pBR322

The vector pBR322 (commercially available, Pharmacia Co.) having ampicillin resistance and tetracycline resistance as markers was completely digested with restriction endonuclease BamH I, and then treated with alkaline phosphatase.

(3) Recombination reaction

In 50 mM tris-HCl buffer (pH 7.4) containing 10 mM $MgCl_2$, 1 mM ATP and 10 mM dithiothreitol, 0.1 $\mu$g of the DNA fragments prepared in (1) above and 0.1 $\mu$g of the pBR322 cleaved in (2) above were mixed with 0.3 unit of T4 DNA ligase, and the resulting mixture was incubated overnight at 4°C, whereby hybrid plasmids composed of pBR322 and the DNA fragments inserted therein were obtained.

(4) Introduction of hybrid plasmids into Escherichia coli

Escherichia coli DH I (see Molecular Cloning A Laboratory Manual, p. 505 (1982)) was incubated with shaking in 100 ml of $\Phi$ culture media (0.5% yeast extract, 2% bactotryptone, 0.5% $MgSO_4$; pH 7.6) at 37°C for about 2.5 hours, and bacterial cells were harvested at the logarithmic growth phase by centrifugation. The bacterial cells were washed with 40 ml of tfb I solution (30 mM potassium acetate, 100 mM RbCl, 10 mM $CaCl_2$, 50 mM $MnCl_2$, 15% glycerol; pH 5.8), and the washed cells were suspended in 10 ml of tbf II solution (10 mM MOPS, 75 mM $CaCl_2$, 10 mM RbCl, 15% glycerol; pH 6.5), after which the resulting suspension was allowed to stand at 0°C for 90 minutes to obtain competent cells.

To 0.2 ml of the competent cell suspension was added 0.1 $\mu$g of the hybrid plasmids obtained in (3) above, and the resulting mixture was allowed to stand at 0°C for 30 minutes. After the mixture was maintained at 42°C for 90 seconds, 0.8 ml of $\Phi$ culture media was added and the resulting mixture was incubated at 37°C for 1 hour. Then, 0.1 ml of this mixture was streaked on 1.5% agar plate of LB culture media containing 100 $\mu$g/ml of ampicillin, and the agar plate was incubated overnight at 37°C.

Subsequently, about 2,500 colonies formed on the plates were picked up with toothpicks and inoculated on 1.5% agar plate of LB culture media containing 15 $\mu$g/ml of tetracycline, and incubated overnight at 37°C. By this procedure, there were obtained about 1,000 ampicillin-resistant, tetracycline-sensitive strains which were able to grow on the culture media containing ampicillin, but not able to grow on the culture media containing tetracycline.

(5) Isolation of hybrid plasmid

The ampicillin-resistant, tetracycline-sensitive strains thus obtained were divided into 5 groups (each group contained about 200 different hybrid plasmids), after which each group was incubated overnight with shaking in 50 ml of LB culture media containing 50 $\mu$g of ampicillin at 37°C, and bacterial cells were harvested by centrifugation. Plasmid DNA was extracted from the bacterial cells by the method of Birnboim and Doly (Nucleic Acid Res., 7, 1513 - (1979)). The plasmid DNAs thus obtained was composed of pBR322 as a vector and various DNA fragments from Bacillus sphaericus FERM BP-1098 inserted therein, and the average chain length of the inserted DNA fragments in this group of hybrid plasmid DNAs was about 6 kb.

(6) Transformation of the biotin auxotrophic E. coli with the hybrid plasmid

The mutant strain Escherichia coli K3 having no biosynthetic ability to produce biotin from desthiobiotin (DTB) was obtained from Escherichia coli C600 by mutagenic treatment according to the method of Pai and Lichstein (J. Bacteriol., 94, 1930 (1967)) by use of N-methyl-N′-nitro-N-nitrosoguanidine. The mutant strain was subjected to shaking culture in 100 ml of LB culture media at 37°C for about 2.5 hours, and bacterial cells were harvested at the logarithmic growth phase by centrifugation. The bacterial cells were made into competent cells by the method of Kushuner (Genetic Engineering, p. 17 (1978)).

next, 0.1 $\mu$g of each of the groups of hybrid plasmid DNAs obtained in (5) above was added to 0.2 ml of a suspension of the above-mentioned competent cells, and the resulting mixture was allowed to stand at 0°C for 30 minutes. After the mixture was maintained at 43.5°C for 30 seconds, 1.8 ml of LB culture media was added and the resulting mixture was incubated at 37°C for 1 hour, after which bacterial cells were harvested by centrifugation. The bacterial cells were washed twice with 0.85% NaCl and then suspended in 1 ml of 0.85% NaCl. Next, 0.1 ml of the resulting cell suspension was streaked on 1.5% agar culture plate of CA culture media (2% glycerol, 1% vitamin-free casamino acid, 0.2% $K_2HPO_4$, 0.1% $KH_2PO_4$, 0.005% $MgSO_4 \cdot 7H_2O$, 0.001% $FeSO_4 \cdot 7H_2O$, 0.001% $MnSO_4 \cdot 4 \sim 6H_2O$, 0.00001% thiamine hydrochloride) containing 50 $\mu$g/ml of ampicillin, and the plates thus prepared were incubated at 37°C for about 36 hours. The two colonies formed were ampicillin-resistant and no-biotin-requiring. Thus, there were obtained two derived strains transformed with the hybrid plasmids having as an insert, DNA coding for genetic information involved in biotin biosynthesis.

(7) Analysis of hybrid plasmid

Each of the two strains of transformants obtained in (6) above was incubated overnight with shaking in 5 ml of CA culture media containing 50 $\mu$g/ml of ampicillin, and bacterial cells were harvested by centrifugation, after which hybrid plasmid DNA was extracted by the method of Birnboim and Doly as described above.

Both hybrid plasmid DNAs obtained from the individual strains had a total chain length of about 12.5 kb, and contained about 8.2 kb of DNA fragments inserted therein. Cleavage patterns of the two hybrid plasmid DNAs were prepared using various restriction endonucleases (BamH I, Hind III, Pst I, EcoR I and Sal I) and compared to find that the two hybrid plasmid DNAs had exactly the same cleavage patterns, indicating that they were identical. It was also confirmed that this hybrid plasmid did not have cleavage sites by Bgl II, Xho I and Kpn I. This hybrid plasmid DNA was named pSB01. The restriction endonuclease cleavage map is shown in Fig. 1 in which the number shows the approximate number of base pairs.

Next, Southern hybridization was performed in order to prove that the DNA fragment inserted into pSB01 plasmid DNA was DNA fragments from Bacillus sphaericus IFO 3525. First, the total DNAs extracted and purified in (1) above were cleaved using restriction endonuclease EcoR I, subjected to agarose gel electrophoresis, and then transferred to nitrocellulose filter by the method of Southern (J. Mol. Biol., 98, 503 (1975)). Separately, 1 $\mu$g of pSB01 plasmid DNA was labeled with $^{32}$P to obtain probe DNA. This prove DNA was prepared by nick translation according to the method of Rigby et al. (J. Mol. Biol., 113, 237 (1977)) and had a specific activity of 114 × $10^6$ cpm/$\mu$g DNA. By use of 0.1 $\mu$g of the probe DNA, hybridization was performed on the previously prepared nitrocellulose filter by the following procedure. First, the filter was treated with a mixture solution obtained by adding 20 $\mu$g/ml of heat-treated salmon sperm DNA to 20 ml of a prehybridization buffer comprising 6 × SSC solution (0.15 M NaCl, 0.015 M sodium citrate) and Denhardt Solution (0.02% bovine serum albumin, 0.02% polyvinyl pyrrolidone, 0.02% Ficoll), and prehybridization was performed at 65°C for 4 hours. Then, to 20 ml of hybridization buffer consisting of 6 × SSC solution, Denhardt solution and 0.5% SDS was added heat-treated salmon sperm DNA so as to adjust its concentration to 50 $\mu$g/ml, followed by adding thereto 0.1 $\mu$g of heat-treated probe DNA, and hybridization was carried out overnight at 67°C. Subsequently, the filter was sufficiently washed with the hybridization buffer, with 6 × SSC solution at 65°C for 30 minutes, and then with 2 × SSC solution at 65°C for 30 minutes. This procedure was repeated twice, after which the washed filter was air-dried and then subjected to autoradiography. As a result, it was confirmed that fragments reactive to the probe DNA existed in the total DNAs. This proved that the approx. 8.2 kb inserted DNA fragments in pSB01 plasmid DNAs were DNA fragments from Bacillus sphaericus IFO 3525.

On the other hand, Southern hybridization was performed with EcoR I cleavage fragments of total DNAs of Bacillus sphaericus IFO 3525 by use of biotin operons of Escherichia coli as probes according to the method of Gibbs et al. (Proc. Natl, Acad. Sci. in USA, 81, 3365 (1984)). In this case, no hybridization occurred even under conditions where hybridization wound have occurred if there had been partial complementarity between the EcoR I cleavage fragments and the biotin operons. From this fact, it was found that the complementarity between biotin operon of Escherichia coli and DNA of Bacillus sphaericus was very slight, so that the inserted DNA fragment of pSB01 was far different from DNA fragment of Escherichia coli.

(8) Confirmation of the reproductivity of transformation

When Escherichia coli K3 was transformed once more with pSB01 plasmid DNA by the method described in (6) above, all Escherichia coli K3 cells containing pSB01 as a plasmid became ampicillin-resistant and no-biotin-requiring. From this fact, it was found that pSB01 plasmid DNA at least had DNA fragment of Bacillus sphaericus IFO 3525 having genetic information capable of making up for the lack of biosynthetic ability to produce biotin from desthiobiotin of Escherichia coli K3.

The Escherichia coli K3 containing this pSB01 plasmid DNA as plasmid was named Escherichia coli K3 (pSB01). This microorganism was deposited to Fermentation Research Institute as FERM BP-1099.

Example 2

(1) Reconstruction of hybrid plasmid

By use of restriction endonuclease EcoR I, 1 μg of the pSB01 plasmid DNA obtained in Example 1 was completely digested, after which 1 μg of the mixture of three kinds DNA fragments thus obtained was mixed with 3 units of T4 DNA ligase in 50 mM tris-HCl buffer (pH 7.4) containing 10 mM $MgCl_2$, 1 mM ATP and 10 mM dithiothreitol, and the resulting mixture was incubated overnight at 4°C to carry out reconstruction.

(2) Transformation of biotin auxotropic Escherichia coli

A transformed strain by hybrid plasmid was obtained in the same manner as in Example 1 (6), except for using 0.1 μg of the above reaction mixture obtained by use of T4 DNA ligase.

(3) Analysis of hybrid plasmid

The transformed strain obtained in (2) above was incubated overnight with shaking in 5 ml of CA culture media containing 50 μg/ml ampicillin, and bacterial cells were harvested by centrifugation, after which hybrid plasmids DNA was extracted from the cells by the method of Birnboim and Doly described above. The smallest plasmid DNA in this extract had a total chain length of about 8.8 kb and corresponded to pSB01 plasmid DNA which had lost EcoR I cleavage fragment of about 3.7 kb. This hybrid plasmid was named pSB03. Its restriction endonuclease cleavage map is shown in Fig. 2.

The Escherichia coli K3 containing this pSB03 plasmid DNA was named Escherichia coli K3 (pSB03). (FERM P-8369).

Example 3

(1) Reconstruction of hybrid plasmid

By use of restriction endonuclease Hind III, 3 μg of the pSB03 obtained in Example 2 was completely digested. Separately, 1 μg of pBR322 was completely digested using restriction endonuclease Hind III. Subsequently, 1 μg of the mixture of three kinds of DNA fragments obtained by the cleavage of pSB03 and 1 μg of the cleaved pBR322 were mixed with 3 units of T4 DNA ligase in 50 mM tris-HCl buffer (pH 7.4) containing 10 mM $MgCl_2$, 1 mM ATP and 10 mM dithiothreitol, to prepare a reaction mixture. This mixture was incubated overnight at 4°C to obtain a hybrid plasmid.

(2) Transformation of biotin auxotrophic Escherichia coli

A transformed strain of hybrid plasmid was obtained in the same manner as in Example 1 (6), except that 1 μg of the above-mentioned reaction mixture was used.

(3) Analysis of hybrid plasmid

The transformed strain obtained in (2) above was incubated overnight with shaking in 5 ml of CA culture media containing 50 $\mu$g/ml of ampicillin, and bacterial cells were harvested by centrifugation, after which hybrid plasmid DNA was extracted by the method of Birnboim and Doly described above. The smallest plasmid DNA in the extract had a total chain length of about 6.2 kb. This plasmid had Hind III cleavage fragment of about 2.0 kb of pSB03 DNA inserted therein, and was named pSB16. Its restriction endonuclease cleavage map is as shown in Fig. 3.

The DNA fragment derived from Bacillus sphaericus IFO 3525, which was included in this inserted fragment, was an approx. 1.5 kb fragement from Mbo I to Hind III. Its precise restriction endonuclease cleavage map is as shown in Fig. 4.

For further investigation of this approx. 1.5 kb DNA fragment, sequence analysis was carried out by the method of Messing et al. using M 13 phage (Gene, 19, 269 (1982), Science 214, 1205 (1981)). The base sequence is as shown in Fig. 5. This sequence has one large open reading frame from the 5'-terminal on the Mbo I side toward the 3'-terminal on the Hind III side. Concretely, 332 codons are linked to the translation initiation codon GTG at the positions 282 to 284. By comparing the base sequence upstream to the translation initiation codon with the sequence of 16 S ribosomal RNA known for Bacillus subtilis, it could be confirmed that the sequence at the positions from 265 to 278 corresponded to Shine-Dalgarno sequence.

The Escherichia coli containing pSB16 plasmid DNA was named Escherichia coli K3 (pSB16).

Example 4

Each of four bacterial strains, i.e., Escherichia coli K3 (pSB01), Escherichia coli K3 (pSB03), Escherichia coli K3 (pSB16) and Escherichia coli C600 (pBR322) containing pBR322 as a plasmid was inoculated into 3 ml of GP culture media (2% glycerol, 3% proteose•peptone, 0.5% vitamin-free casamino acid, 0.1% $K_2HPO_4$, 0.05% KCl, 0.05% $MgSO_4$•$7H_2O$, 0.001% $MnSO_4$•4 ~ $6H_2O$, 0.001% $FeSO_4$•$7H_2O$, 0.000002% thiamine hydrochloride; pH 7.0) containing 50 $\mu$g/ml of ampicillin and 10 $\mu$g/ml of DL-desthiobiotin, and the resulting mixture was incubated with shaking at 37°C for 4 days.

After the incubation, 1 ml of the culture broth was centrifuged to precipitate bacterial cells, and the supernatant was transferred to a test tube. Then, the supernatant was treated at 120°C for 30 minutes, after which the amount of biotin accumulated in the supernatant was determined by microbiological assay using Lactobacillus plantarum ATCC 8014. The amount of biotin accumulated by each strain is shown in Table 1.

## Table 1

| Name of microorganism | Accumulation amount of biotin (ng/ml-culture media) |
|---|---|
| Escherichia coli K3 (pSB01) | 740 |
| Escherichia coli K3 (pSB03) | 1217 |
| Escherichia coli K3 (pSB16) | 655 |
| Escherichia coli C600 (pBR322) | 0 |

From the results shown in Table 1, it is evident that the microorganisms of this invention accumulate a larger amount of biotin in culture media than does Escherichia coli C600 (pBR322).

Example 5

(1) Construction of hybrid plasmid pSB103

An experiment was carried out in the same manner as in Example 3, except that pUC9 (commercially available, Pharmacia Co.) having an ampicillin resistance as a marker was used in place of pBR322, to obtain a hybrid plasmid (pSB103) which had a total chain length of about 4.6 kb and contained an approx. 2.0 kb DNA fragment inserted therein as in the case of pSB16 (see Fig. 6). Escherichia coli K3 transformed by this pSB103 was named Escherichia coli K3 (pSB103).

(2) Construction of hybrid plasmid pSB301 (see Fig. 6)

The following procedure was carried out in order to link biotin synthetase gene (bioB gene) directly to the promoter operator region of trp operon. pSB103 was completely digested with restriction endonuclease Mae I, after which 2.0 $\mu$g of the DNA fragments obtained by the digestion were mixed with 1 unit of Sl nuclease in a mixture of 30 mM sodium acetate (pH 4.5), 250 mM NaCl, 1 mM ZnSO$_4$ and 5% glycerol, and the resulting mixture was incubated at 37°C for 30 minutes, whereby both ends of the DNA fragments were blunted. Subsequently, the reaction mixture was subjected to agarose gel electrophoresis and approx. 2.1 kb DNA fragments were recovered.

On the other hand, pDR720 (commercially available, Pharmacia Co.) containing the promoter operator region of trp operon and ampicillin resistance as a marker was completely digested with restriction endonuclease Sma I, and then treated with alkaline phosphatase.

With 2 units of T4 DNA ligase were mixed 0.1 $\mu$g of the approx. 2.1 kb DNA fragment recovered from the agarose gel and 0.1 $\mu$g of the cleaved pDR720 in 50 mM tris-HCl buffer (pH 7.4) containing 10 mM MgCl$_2$, 1 mM ATP and 10 mM dithiothreitol, and the resulting mixture was incubated at 15°C for 4 hours to obtain a reaction mixture containing a hybrid plasmid.

A transformed strain by the hybrid plasmid was obtained in the same manner as in Example 1 (6) except for using 0.2 $\mu$g of this reaction mixture.

The transformant thus obtained was subjected to shaking culture overnight in 3 ml of LB media containing 50 $\mu$g/ml of ampicillin, and bacterial cells were harvested by centrifugation, after which hybrid plasmid DNA was extracted from the cells by the method of Birnboim and Doly. The smallest plasmid DNA in this extract had a total chain length of about 6.1 kb. In this plasmid, the translation initiation codon GTG of biotin synthetase gene derived from Bacillus sphaericus started from about 70 bases downstream to the transcription initiation site of trp promoter. This plasmid was named pSB301.

Escherichia coli MC 169 (J. Bacteriol., 143, 852 (1980)) was transformed with pSB301 according to the method of Mandel and Higa (J. Mol. Biol., $\overline{53}$, 154, (1970)), and the transformant thus obtained was named Escherichia coli MC 169 (pSB301).

Example 6

The following procedure was carried out in order to link biotin synthetase gene directly to the promoter region of $\alpha$-amylase gene of Bacillus licheniformis.

(1) Construction of pBHB10

pSB103 was completely digested with restriction endonuclease Hind III, after which the reaction mixture was subjected to agarose gel electrophoresis and approx. 2.0 kb DNA fragments were recovered. Restriction endonuclease Mae III was added to 2.0 $\mu$g of these DNA fragments, and the resulting mixture was incubated at 45°C for 10 minutes to cleave the DNA fragments partially. Then, the reaction mixture was subjected to agarose gel electrophoresis and approx. 1.2 kb DNA fragments were recovered.

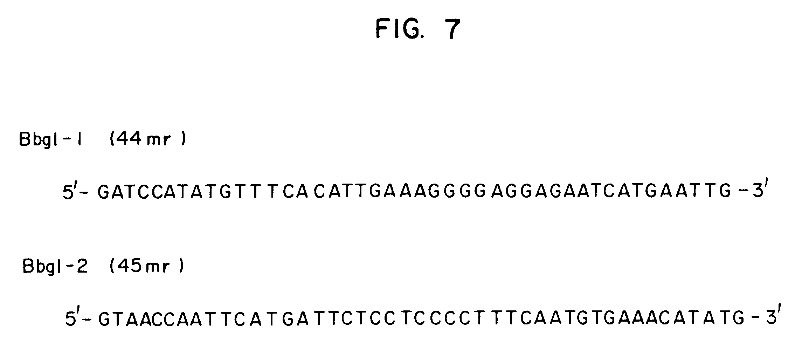

On the other hand, the oligonucleotides, fragments Bbgl-1 and Bbgl-2 shown in Fig. 7 were obtained by means of a full automatic synthesizer (Genet A-III, mfd. by Nippon Zeon Co., Ltd.). Of these fragments, 1 $\mu$g of Bbgl-1 fragment was incubated with ATP and 1 unit of T4 polynucleotide kinase at 37°C for 30 minutes in a mixture of 50 mM tris-HCl buffer (pH 7.6), 10 mM MgCl$_2$, 5 mM dithiothreitol and 0.1 mM spermidine, to phospholylate the hydroxyl group at the 5′-terminal. Then, 1 $\mu$g of Bbgl-2 fragment was added to conduct annealing.

Subsequently, pUC18 (commercially available, Pharmacia Co.) having ampicillin resistance as a marker was completely digested with endonuclease BamH I and Hind III, and then treated with alkaline phosphatase.

With 2 units of T4 DNA ligase was mixed 1 µg of approx. 2.7 kb DNA fragments of the cleaved pUC18 and the synthetic fragments previously obtained by the annealing, in 50 mM tris-HCl buffer (pH 7.4) containing 10 mM MgCl₂, 1 mM ATP and 10 mM dithiothreitol. The resulting mixture was incubated at 15°C for 2 hours, after which the reaction mixture was subjected to agarose gel electrophoresis and approx. 2.7 kb DNA fragments were recovered. Subsequently, 0.4 µg of the recovered DNA fragments and 0.015 µg of the previously prepared approx. 1.2 kb DNA fragments were mixed with 2 units of T4 DNA ligase in a buffer solution having the same composition as described above, and the resulting mixture was incubated at 15°C for 3 hours.

Transformed strains by hybrid plasmid were obtained in the same manner as in Example 1 (6) except for using this reaction mixture.

Each of the transformants thus obtained was subjected to shaking culture overnight in 3 ml of LB media containing 50 µg/ml of ampicillin, and bacterial cells were harvested by centrifugation, after which hybrid plasmid DNA was extracted from the cells by the method of Birnboim and Doly.

Each of the plasmid DNAs extracted was completely digested with restriction endonuclease BamH I and Hind III and then subjected to agarose gel electrophoresis to obtain approx. 4.0 kb plasmid DNA composes of approx. 1.3 kb fragment containing the synthetic DNA and the approx. 1.2 kb DNA fragment prepared from pSB103 derived from Bacillus sphaericus and the approx. 2.7 kb DNA fragment derived from pUC18. This plasmid DNA was named pBHB10.

## (2) Construction of pUC18-AP

pUC18 was completely digested with restriction endonuclease Nde I, after which 2.0 µg of the DNA fragments obtained by this cleavage were mixed with 1 unit of SI nuclease in a mixture of 30 mM sodium acetate (pH 4.5), 250 mM NaCl, 1 mM ZnSO₄ and 5% glycerol, and the mixture thus obtained was incubated at 37°C for 30 minutes, whereby both ends of the DNA fragments were blunted. Subsequently, 1.0 µg of the reaction mixture was mixed with 5 units of T4 DNA ligase in 50 mM tris-HCl buffer (pH 7.4) containing 10 mM MgCl₂, 1 mM ATP and 10 mM dithiothreitol, and the mixture thus obtained was incubated overnight at 4°C to carry out re-cyclization.

Escherichia coli C600 was transformed using the thus obtained reaction mixture according to the method of Mandel and Higa. The transformant thus obtained was grown on LB agar culture media containing 50 µg/ml ampicillin. Plasmid DNA extracted from the grown transformant was an approx. 2.7 kb plasmid corresponding to pUC18 which had lost Nde I cleavage site. This plasmid DNA was named pUC18-N.

This pUC18-N was completely digested with restriction endonucleases EcoR I and Pst I, and the completed digested DNA fragments were mixed with 1 unit of T4 DNA ligase and approx. 1.1 kb DNA fragment containing α-amylase gene promoter region of Bacillus licheniformis obtained by EcoR I - Pst I cleavage in 50 mM tris-HCl buffer (pH 7.4) containing 10 mM MgCl₂, 1 mM ATP and 10 mM dithiothreitol. The mixture thus obtained was incubated at 15°C for 2 hours.

Escherichia coli C600 was transformed using the reaction mixture according to the method of Mandel and Higa. The transformed strain was grown on LB agar culture media containing 50 µg/ml of ampicillin, and then subjected to shaking culture overnight in 3 ml of LB culture media containing 50 µg/ml of ampicillin, and bacterial cells were harvested by centrifugation. Then, plasmid DNA was extracted from the cells by the methods of Birnboim and Doly. Each extracted plasmid DNA was completely digested with restriction endonuclease EcoR I and Pst I, and then subjected to agarose gel electrophoresis to obtain approx. 3.8 kb plasmid DNA composed of approx. 1.1 kb DNA fragment derived from Bacillus licheniformis and approx. 2.7 kb DNA fragment derived from pUC18-N. This plasmid DNA was named pUC18-AP.

## (3) Construction of pBHB11

pBHB10 was completely digested with restriction endonuclease Hind III, after which restriction endonuclease Nde I was added to 6.0 µg of the DNA fragments obtained by this cleavage, and the resulting mixture was incubated at 37°C for 5 minutes to cleave the DNA fragments partially. Subsequently, the reaction mixture was subjected to agarose gel electrophoresis and approx. 1.3 kb DNA fragments were recovered.

With 1 unit of T4 DNA ligase were mixed 0.1 µg of the recovered DNA fragments and DNA fragments obtained by completely digesting pUC18-AP with restriction endonucleases Nde I and Hind III, in 50 mM tris-HCl buffer (pH 7.4) containing 10 mM MgCl₂, 1 mM ATP and 10 mM dithiothreitol, and the mixture thus obtained was incubated at 15°C for 2 hours.

Escherichia coli C600 was transformed using the thus obtained reaction mixture according to the method of Mandel and Higa. The transformed strain was grown on LB agar culture media containing 50 $\mu$g/ml of ampicillin, and then subjected to shaking culture overnight in 3 ml of LB culture media containing 50 $\mu$g/ml of ampicillin, and bacterial cells were harvested by centrifugation. Then, plasmid DNA was extracted from the cells by the method of Birnboim and Doly. Each extracted plasmid DNA was completely digested with restriction endonucleases EcoR I and Hind III, and then subjected to agarose gel electrophoresis to obtain approx. 5.0 kb plasmid DNA composed of approx. 2.4 kb DNA fragment containing the promoter region of $\alpha$-amylase gene and biotin synthetase gene and approx. 2.6 kb DNA fragment derived from pUC18-N. This plasmid DNA was named pBHB11.

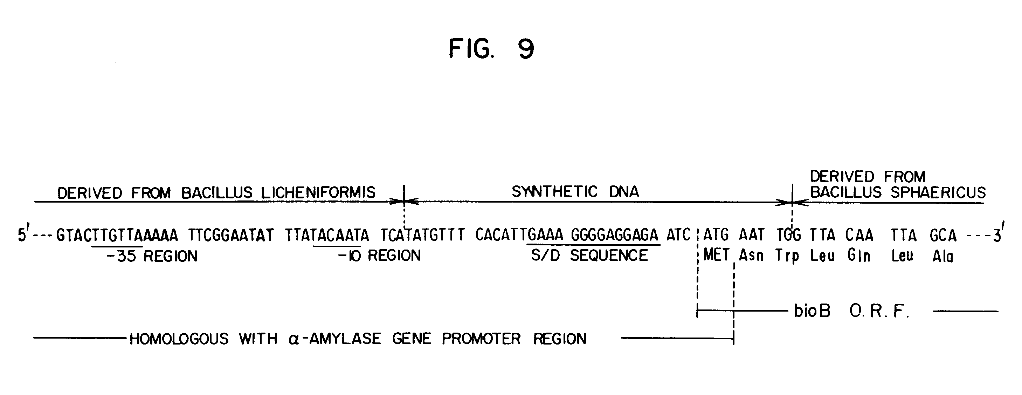

The sequence analysis of the DNA was carried out mainly for the binding site of the promoter region of $\alpha$-amylase gene and the biotin synthetase gene by the method of Messing et al. using M13 phage. The base sequence was as shown in Fig. 9. It can be understood that the sequence of the open reading frame (O.R.F) coding for biotin synthetase of Bacillus sphaericus from which the translation initiation codon GTG has been removed is linked to the $\alpha$-amylase gene promoter sequence Shine-Dalgarno (SD) sequence and translation initiation codon ATG of Bacillus licheniformis.

### (4) Construction of pBHB13

pBHB11 and pUB110 (J. Bacteriol. 134, p318 (1978) which was a plasmid replicable in bacteria of the genus Bacillus and had kanamycin resistance as a marker, were completely digested with restriction endonuclease EcoR I. Then, 0.2 $\mu$g of the cleaved pBHB10 and 0.2 $\mu$g of the cleaved pUB110 were mixed with 2 units of T4 DNA ligase in 50 mM tris-HCl buffer containing 10 mM MgCl$_2$, 1 mM ATP and 10 mM dithiothreitol, and the mixture thus obtained was incubated at 15°C for 2 hours.

Escherichia coli C600 was transformed using the reaction mixture according to the method of Mandel and Higa. The transformed strain was grown on LB agar culture media containing 50 $\mu$g/ml of ampicillin and about 400 colonies formed thereon were subjected to replica on LB agar culture media containing 10 $\mu$g/ml of kanamycin, to obtain 52 strains of transformants having ampicillin resistance and kanamycin resistance (km$^r$). Each of these transformants was subjected to shaking culture overnight in 3 ml of LB culture media containing 10 $\mu$g/ml of kanamycin, and bacterial cells were harvested by centrifugation. Then, plasmid DNA was extracted from the cell by the method of Birnboim and Doly. The smallest of the plasmid DNAs thus extracted had a total chain length of about 9.5 kb. This plasmid was a hybrid plasmid composed of pBHB11 and pUB110 and was a shuttle vector which was replicable either in bacteria of the genus Escherichia or in bacteria of the genus Bacillus. This plasmid was named pBHB13 (see Fig. 8).

### (5) Transformation of Bacillus sphaericus

Bacillus sphaericus NCIB 9370 was subjected to shaking culture in 50 ml of PB culture medium (Difco antibiotic medium No. 3, 1.75%) at 37°C until the intermediate stage of logarithmic growth phase, and bacterial cells were havested by centrifugation. The cells were suspended in 5 ml of a solution prepared by adding 1% of BSA to SMMP solution [a mixture of 2 × SMM (1 M sucrose, 40 mM sodium malate, 40 mM MgCl$_2$•6H$_2$O; pH 6.5) and an equal amount of 4 × PB (Difco abtibiotic medium No. 3, 7%)], and acetylmuramidase was added so as to adjust its concentration to 0.3 mg/ml. Then, the resulting mixture was incubated at 37°C for 45 minutes. Bacterial cells were harvested by centrifugation, washed twice with SMMP, and then suspended in 1 ml of SMMP to obtain a protoplast suspension.

To 0.5 ml of the protoplast suspension was added 1 $\mu$g of pBHB 13 prepared from Escherichia coli, followed by adding thereto 1.5 ml of 40% PEG 6000 (a mixture prepared by adding 50 ml of 2 × SMM to 40 g of polyethylene glycol (6000), followed by adding distilled water to make up to 100 ml), and gentle suspending was conducted. Then, the resulting suspension was allowed to stand at room temperature for 2 minutes and then diluted with 5 ml of SMMP, after which bacterial cells were harvested. The cells were suspended in 1 ml of SMMP containing 1% of BSA and subjected to stationary culture at 30°C for 1.5 hours, after which 0.1 ml of the culture broth was streaked on DM 4 medium (0.8% agar, 0.5% casamino acid, 0.5% yeast extract, 0.33 M succinic acid (pH 7.3), 0.35% K$_2$HPO$_4$, 0.15% KH$_2$PO$_4$, 0.5% glycerol, 0.02 M MgCl$_2$, 0.01% BSA) containing 150 $\mu$g/ml of kanamycin, and this medium was incubated at 37°C for 3 days to 1 week.

Subsequently, colonies formed on the medium were picked up with toothpicks and inoculated into GP culture media containing 10 $\mu$g/ml of kanamycin, after which this media was incubated overnight at 37°C, and bacterial cells were harvested by centrifugation. Plasmid DNA was extracted from the cells by the method of Birnboim and Doly. The restriction endonuclease cleavage pattern of the plasmid DNA thus obtained was investigated and found to be same as that of pBHB 13 extracted from Escherichia coli. From this results, it was confirmed that pBHB 13 had been inserted into Bacillus sphaericus NCIB 9370. However, the restriction endonuclease BamH I cleavage site present on this pBHB13 was not cleaved by restriction endonuclease BamH I because it had been modified by methylase in Bacillus sphaericus NCIB 9370 cells. The Bacillus sphaericus NCIB 9370 transformed by pBHB13 was named as Bacillus sphaericus NCIB 9370 (pBHB13).

In addition, a transformant obtained in the same manner as described above, except that Bacillus sphaericus IFO 3525 was used in place of Bacillus sphaericus NCIB 9370 and transformed with pBHB13 extracted from Bacillus sphaericus NCIB 9370 (pBHB13), was named Bacillus sphaericus IFO 3525 (pBHB13).

Similarly, Bacillus sphaericus NCIB 9370 and Bacillus sphaericus IFO 3525 which had been transformed by pUB110, were named Bacillus sphaericus NCIB 9370 (pUB110) and Bacillus sphaericus IFO 3525 (pUB110).

Example 7

Each of the two strains, Escherichia coli MC 169 (pSB301) and Escherichia coli MC 169 (pDR720) having pDR720 as a plasmid was inoculated into 3 ml of a 1.2-fold concentration of PC cultre medium (2% glycerol, 5% proteose•peptone, 2% casamino acid, 0.1% $K_2HPO_4$, 0.05% KCl, 0.05% $MgSO_4 \cdot 7H_2O$, 0.001% $MnSO_4 \cdot 4 \sim 6H_2O$, 0.001% $FeSO_4 \cdot 7H_2O$; pH 7.0) containing 50 $\mu$g/ml ampicillin and 100 $\mu$g/ml of DL-desthiobiotin (DTB), and subjected to shaking culture at 37°C for 1 hour. Then, in order to induce trp promoter, indoleacrylic acid was added so as to adjust its concentration to 16.7 $\mu$g/ml, and shaking culture was carried out overnight at 37°C.

After the culture, 1 ml of the culture broth was centrifuged to precipitate bacterial cells, and the supernatant was transfered to a test tube. Subsequently, the supernatant was treated at 120°C for 30 minutes, after which the amount of biotin accumulated in the supernatant was determined by microbiological assay using Lactobacillus plantarum. The amount of biotin accumulated by each strain is shown in Table 2.

## Table 2

| Name of microorganisms | Accumulation amount of biotin ($\mu$g/ml-culture media) |
|---|---|
| Escherichia coli MC 169 (pSB301) | 30 |
| Escherichia coli MC 169 (pDR720) | 0 |

From the results shown in Table 2, it is evident that the microorganisms of this invention accumulate a larger amount of biotin in culture media than does Escherichia coli MC 169 (pDR720).

In addition, the accumulation amount of biotin of the microorganism according to this invention is larger than that of various biotin highly producing mutant strains (e.g., Japanese Patent Application Kokai (Laid-Open) Nos. '86-149091 and '86-202686). From this fact, it is possible to understand the valuability of the gene coding for biotin synthetase derived from a microorganism of the genus Bacillus.

Example 8

Each of the four strains, Bacillus sphaericus NCIB 9370 (pBHB13), Bacillus sphaericus NCIB 9370 (pUB110), Bacillus sphaericus IFO 3525 (pBHB13) and Bacillus sphaericus IFO 3525 (pUB110) was inoculated into 3 ml of GP culture media and subjected to shaking culture overnight at 28°C. Then, at the last stage of logarithmic growth phase, there was added either DL-DTB so as to adjust its concentration to 25 μg/ml or pimelic acid so as to adjust its concentration to 500 μg/ml, and shaking of the culture was carried out at 37°C for 2 days.

After the culture, the culture supernatant was subjected to determination of accumulation amount of biotin by microbiological assay using Lactobacillus plantarum. In Table 3 are shown the amount of biotin accumulated by each strain by use of each precursor.

## Table 3

| | Name of micro-organisms | Accumulation amount of biotin (μg/ml-culture media) | |
|---|---|---|---|
| | | Precursor | |
| | | DL-DTB | Pimelic acid |
| Example of the Invention | Bacillus sphaericus NCIB 9370 (pBHB13) | Unmeasured | 1.1 |
| Reference Example | Bacillus sphaericus NCIB 9370 (pUB110) | Unmeasured | 0.03 |
| Example of the Invention | Bacillus sphaericus IFO 3525 (pBHB13) | 3.0 | 4.8 |
| Reference Example | Bacillus sphaericus IFO 3525 (pUB110) | 0.16 | 0.18 |

From the results shown in Table 3, it can be understood that the microorganisms of this invention produce biotin by use of pimelic acid as a precursor, as in the case of using DL-desthiobiotin as a precursor.

## Claims

1. A nucleotide sequence which codes for a polypeptide which comprises part or all of an amino acid sequence shown in Fig.5 or a derivative thereof, and which has the capability of catalysing the biosynthetic conversion of desthiobiotin to biotin.

2. A nucleotide sequence according to Claim 1, which codes for a polypeptide which comprises the amino acid sequence from position 285 to position 1277 in Fig.5, or a functional derivative thereof.

3. A nucleotide sequence according to Claim 1 which codes for a polypeptide which comprises the amino acid sequence from position 265 to position 1280 in Fig.5 or a functional derivative thereof.

4. A nucleotide sequence according to Claim 1, Claim 2 or Claim 3 which has a translation initiation codon upstream thereof and a translation termination codon downstream thereof.

5. A nucleotide sequence according to any one of Claims 1 to 4 which has a Shine-Dalgarno sequence and a translation initiation codon both upstream thereof and a translation termination codon downstream thereof.

6. A nucleotide sequence according to Claim 5, wherein said Shine-Dalgarno sequence is GAAAAGGAGAATGT.

7. A recombinant vector containing, as an insert, a nucleotide sequence according to any one of Claims 1 to 6.

8. A recombinant vector according to Claim 7, wherein said nucleotide sequence is inserted under the control of a promoter.

9. A recombinant vector according to Claim 8, wherein said promoter functions in bacteria of the genus Escherichia, Bacillus or Pseudomonas.

10. A recombinant vector according to any one of Claims 7 to 9 wherein the vector is a plasmid.

11. A recombinant vector according to any one of Claims 7 to 9 wherein the vector is a hybrid plasmid.

12. A bacterium transformed or transduced with a recombinant vector according to any one of Claims 7 to 11.

13. A bacterium according to Claim 12, which belongs to the genus Escherichia, Bacillus or Pseudomonas.

14. A bacterium according to Claim 13 which is of the strain Bacillus sphaericus.

15. A bacterium according to any one of Claims 12 to 14 which has desthiobiotin productivity.

16. A process for producing biotin which comprises culturing a bacterium according to any one of Claims 12 to 15 in a culture medium containing a suitable carbon source in the presence of a precursor for biotin biosynthesis, to allow the bacterium to produce biotin, and
separating and isolating the biotin thus produced.

17. A process according to Claim 16 wherein said precursor is desthiobiotin.

18. A process according to Claim 16 wherein said precursor is pimelic acid or azelaic acid and said bacterium transformed or transduced has desthiobiotin productivity.

**Patentansprüche**

1. Nucleotidsequenz, die ein Polypeptid codiert, welches einen Teil der oder die gesamte in Fig. 5 gezeigte Aminosäuresequenz umfaßt, oder ein Derivat davon, und die zur Katalyse der biosynthetischen Umwandlung von Disthiobiotin in Biotin fähig ist.

2. Nucleotidsequenz nach Anspruch 1, die ein Polypeptid codiert, welches die Aminosäuresequenz von Position 285 bis Position 1277 in Fig. 5 umfaßt, oder ein funktionelles Derivat davon.

3. Nucleotidsequenz nach Anspruch 1, die ein Polypeptid codiert, welches die Aminosäuresequenz von Position 265 bis Position 1280 in Fig. 5 umfaßt, oder ein funktionelles Derivat davon.

4. Nucleotidsequenz nach Anspruch 1, 2 oder 3, die ein Translations-Initiationscodon stromaufwärts davon und ein Translations-Terminationscodon stromabwärts davon umfaßt.

5. Nucleotidsequenz nach einem der Ansprüche 1 bis 4, die eine Shine-Dalgarno-Sequenz und ein Translations-Initiationscodon, die beide stromaufwärts davon liegen, und ein Translations-Terminationscodon stromabwärts davon umfaßt.

17

EP 0 240 105 B1

**6.** Nucleotidsequenz nach Anspruch 5, wobei die Shine-Dalgarno-Sequenz GAAAAGGAGAATGT ist.

**7.** Rekombinanter Vektor, der als ein Insert eine Nucleotidsequenz nach einem der Ansprüche 1 bis 6 umfaßt.

**8.** Rekombinanter Vektor nach Anspruch 7, wobei die Nucleotidsequenz unter der Kontrolle eines Promotors eingebaut ist.

**9.** Rekombinanter Vektor nach Anspruch 8, wobei der Promotor in Bakterien der Gattung Escherichia, Bacillus oder Pseudomonas wirksam ist.

**10.** Rekombinanter Vektor nach einem der Ansprüche 7 bis 9, wobei der Vektor ein Plasmid ist.

**11.** Rekombinanter Vektor nach einem der Ansprüche 7 bis 9, wobei der Vektor ein Hybridplasmid ist.

**12.** Bakterium, das mit einem rekombinanten Vektor nach einem der Ansprüche 7 bis 11 transformiert oder transduziert ist.

**13.** Bakterium nach Anspruch 12, das zu der Gattung Escherichia, Bacillus oder Pseudomonas gehört.

**14.** Bakterium nach Anspruch 13, das vom Stamm Bacillus sphaericus ist.

**15.** Bakterium nach einem der Ansprüche 12 bis 14, das eine Desthiobiotin-Produktivität aufweist.

**16.** Verfahren zur Erzeugung von Biotin, umfassend die Züchtung eines Bakteriums nach einem der Ansprüche 12 bis 15 in einem Kulturmedium, das eine geeignete Kohlenstoffquelle enthält, in Gegenwart eines Vorläufers für die Biotinbiosynthese, so daß das Bakterium Biotin produzieren kann, und die Abtrennung und Isolierung des so produzierten Biotins.

**17.** Verfahren nach Anspruch 16, wobei der Vorläufer Desthiobiotin ist.

**18.** Verfahren nach Anspruch 16, wobei der Vorläufer Pimelinsäure oder Azelainsäure ist und das transformierte oder transduzierte Bakterium Desthiobiotin-Produktivität aufweist.

**Revendications**

**1.** Séquence de nucléotides codant un polypeptide qui comprend tout ou partie de la séquence d'acides aminés représentée sur la figure 5, ou son dérivé, et qui possède la capacité de catalyser la conversion biosynthétique de la déthiobiotine en biotine.

**2.** Séquence de nucléotides conforme à la revendication 1, qui code un polypeptide comprenant la séquence d'acides aminés allant de la position 285 à la position 1277 sur la figure 5, ou son dérivé fonctionnel.

**3.** Séquence de nucléotides conforme à la revendication 1, qui code un polypeptide comprenant la séquence d'acides aminés allant de la position 265 à la position 1280 sur la figure 5, ou son dérivé fonctionnel.

**4.** Séquence de nucléotides conforme à la revendication 1, 2 ou 3, qui possède un codon de début de traduction situé en amont et un codon de fin de traduction situé en aval.

**5.** Séquence de nucléotides conforme à l'une quelconque des revendications 1 à 4, qui possède une séquence de Shine-Dalgarno et un codon de début de traduction, tous deux situés en amont, et un codon de fin de traduction situé en aval.

**6.** Séquence de nucléotides conforme à la revendication 5, dans laquelle ladite séquence de Shine-Dalgarno est GAAAAGGAGAATGT.

18

**7.** Vecteur recombinant dans lequel a été insérée une séquence de nucléotides conforme à l'une quelconque des revendications 1 à 6.

**8.** Vecteur recombinant conforme à la revendication 7, dans lequel ladite séquence de nucléotides est insérée sous le contrôle d'un promoteur.

**9.** Vecteur recombinant conforme à la revendication 8, dans lequel ledit promoteur fonctionne dans des bactéries du genre Escherichia, Bacillus ou Pseudomonas.

**10.** Vecteur recombinant conforme à l'une quelconque des revendications 7 à 9, dans lequel le vecteur est un plasmide.

**11.** Vecteur recombinant conforme à l'une quelconque des revendications 7 à 9, dans lequel le vecteur est un plasmide hybride.

**12.** Bactérie ayant subi une transformation ou une transduction avec un vecteur recombinant conforme à l'une quelconque des revendications 7 à 11.

**13.** Bactérie conforme à la revendication 12, qui appartient au genre Escherichia, Bacillus ou Pseudomonas.

**14.** Bactérie conforme à la revendication 13, qui est de la souche Bacillus sphaericus.

**15.** Bactérie conforme à l'une quelconque des revendications 12 à 14, qui peut produire de la déthiobiotine.

**16.** Procédé de production de biotine, qui comporte le fait de cultiver une bactérie conforme à l'une quelconque des revendications 12 à 15, dans un milieu de culture contenant une source appropriée de carbone et en présence d'un précurseur pour biosynthèse de biotine, de façon à laisser la bactérie produire de la biotine, et le fait de séparer et d'isoler la biotine ainsi produite.

**17.** Procédé conforme à la revendication 16, dans lequel ledit précurseur est de la déthiobiotine.

**18.** Procédé conforme à la revendication 16, dans lequel ledit précurseur est de l'acide pimélique ou de l'acide azélaïque, et ladite bactérie ayant subi une transformation ou une transduction peut produire de la déthiobiotine.

FIG. 2

FIG. 1

# FIG. 3

# FIG. 4

# FIG. 5A

EP 0 240 105 B1

MboI
5′-GATCGAGTAGAATCACCAACGAAAAAGCATTTCATTGTTGC

42                        72                        102                       132
CAATATTATAGGGCTTATCATTATTTATGCAGTCGCAGTACCTTATTTATATGTAGCATTAAATGTATGGTTAAACATGAAATCAAGTTGGTCTCATGTATTTTTAGTAGGCTTTGTCAA

162                       192                       222                       252
TAGTATTGTTGCAGACTTTTGCTTAGCAATTGCTTCTGCCCTTTTAGCTGAACGTCTATACAAAGTATTCCGTTCCGCTAGAGCTATAAAACTTGTGCAAATTGAAAAGGAGAATGTTTA

282                                    312                                    342
GTG AAT TGG TTA CAA TTA GCA GAT GAA GTG ATT GCA GGC AAG GTA ATT AGC GAT GAT GAG GCA CTT GCC ATT TTA AAT AGT GAT GAT GAT
Asn Trp Leu Gln Leu Ala Asp Glu Val Ile Ala Gly Lys Val Ile Ser Asp Asp Glu Ala Leu Ala Ile Leu Asn Ser Asp Asp Asp

372                                    402                                    432
GAT ATT TTA AAG CTA ATG GAC GGC GCA TTT GCC ATT CGT AAG CAC TAT TAC GGT AAA AAA GTA AAG TTA AAT ATG ATT ATG AAT GCT AAA
Asp Ile Leu Lys Leu Met Asp Gly Ala Phe Ala Ile Arg Lys His Tyr Tyr Gly Lys Lys Val Lys Leu Asn Met Ile Met Asn Ala Lys

462                                    492                                    522
AGT GGC TAT TGC CCA GAG GAT TGT GGC TAT TGC TCG CAG TCA TCT AAA TCG ACC GCT CCT ATT GAG AAA TAT CCG TTC ATT ACA AAA GAA
Ser Gly Tyr Cys Pro Glu Asp Cys Gly Tyr Cys Ser Gln Ser Ser Lys Ser Thr Ala Pro Ile Glu Lys Tyr Pro Phe Ile Thr Lys Glu

552                                    582                                    612
GAA ATA TTA GCG GGG GCA AAG CGT GCG TTT GAA AAT AAA ATT GGT ACG TAT TGC ATC GTC GCA AGC GGA CGT GGG CCG ACT CGT AAA GAT
Glu Ile Leu Ala Gly Ala Lys Arg Ala Phe Glu Asn Lys Ile Gly Thr Tyr Cys Ile Val Ala Ser Gly Arg Gly Pro Thr Arg Lys Asp

642                                    672                                    702
GTC AAT GTA GTG AGT GAA GCC GTT GAA GAA ATT AAA GCA AAA TAT GGC TTA AAA GTT TGC GCT TGC TTA GGT TTA CTA AAA GAA GAA CAA
Val Asn Val Val Ser Glu Ala Val Glu Glu Ile Lys Ala Lys Tyr Gly Leu Lys Val Cys Ala Cys Leu Gly Leu Leu Lys Glu Glu Gln

732                                    762                                    792
GCA CAA CAA TTA AAA GAA GCG GGT GTT GAT CGC TAC AAT CAT AAC TTA AAT ACA TCA GAG CGT CAC CAT TCC TAT ATT ACG ACG ACG CAC
Ala Gln Gln Leu Lys Glu Ala Gly Val Asp Arg Tyr Asn His Asn Leu Asn Thr Ser Glu Arg His His Ser Tyr Ile Thr Thr Thr His

# FIG. 5B

822                                             852                                             882
ACA TAT GAG GAT CGT GTT AAT ACC GTT GAG GTT GTA AAG AAA CAT GGT ATT TCC CCA TGT TCT GGA GCC ATT ATT GGG ATG AAA GAA ACG
Thr Tyr Glu Asp Arg Val Asn Thr Val Glu Val Val Lys Lys His Gly Ile Ser Pro Cys Ser Gly Ala Ile Ile Gly Met Lys Glu Thr

912                                             942                                             972
AAA ATG GAT GTC GTG GAA ATT GCA CGC GCA TTG CAT CAG TTG GAC GCG GAT TCA ATT CCA GTT AAC TTC TTA CAT GCA ATT GAT GGA ACG
Lys Met Asp Val Val Glu Ile Ala Arg Ala Leu His Gln Leu Asp Ala Asp Ser Ile Pro Val Asn Phe Leu His Ala Ile Asp Gly Thr

1002                                            1032                                            1062
AAA CTT GAA GGA ACA CAG GAC TTA AAT CCT CGC TAT TGC TTA AAA GTA TTA GCG TTA TTC CGC TAC ATG AAT CCT TCG AAG GAA ATT AGA
Lys Leu Glu Gly Thr Gln Asp Leu Asn Pro Arg Tyr Cys Leu Lys Val Leu Ala Leu Phe Arg Tyr Met Asn Pro Ser Lys Glu Ile Arg

1092                                            1122                                            1152
ATT TCC GGT GGT CGC GAA GTC AAT TTA GGA TTC CTT CAG CCA TTT GGA CTG TAT GCA GCA AAT AGT ATT TTT GTT GGG GAT TAC TTA ACT
Ile Ser Gly Gly Arg Glu Val Asn Leu Gly Phe Leu Gln Pro Phe Gly Leu Tyr Ala Ala Asn Ser Ile Phe Val Gly Asp Tyr Leu Thr

1182                                            1212                                            1242
ACT GAA GGA CAA GAA GCC AAT AGC GAT TAT CGT ATG CTT GAA GAT TTG GGC TTT GAA ATC GAG CTG ACA CAA AAG CAA GAA GAA GCA TTT
Thr Glu Gly Gln Glu Ala Asn Ser Asp Tyr Arg Met Leu Glu Asp Leu Gly Phe Glu Ile Glu Leu Thr Gln Lys Gln Glu Glu Ala Phe

1272                                            1302                                            1332
TGT TCT TAA TTC AAC CAA TCA TTA TGA AAT AAA ATC TAC TAC TAC ACA ATA TGA TTA CCT CAA AAC CGT GTG AGC GTC GTG GAA AAG GCG
Cys Ser

1362                           1392                           1422                           1452
CACAGACGGTTTTTTGGTCGATAAAAGAGAAGGAGAAAGGTAAATAAATGGTTCCGATAATATACCTATAAAATGATGGTTTTCACAAAATGTTCAATGAAAGCGTTTTGAAATTGAACA

1482                         Hind II
GTTTGTGAAGGGCTTCACATAAAGCTT – 3'

EP 0 240 105 B1

# FIG. 6

# FIG. 7

Bbgl - I   (44 mr )

5′- GATCCATATGTTTCACATTGAAAGGGGAGGAGAATCATGAATTG -3′

Bbgl -2   (45 mr )

5′- GTAACCAATTCATGATTCTCCTCCCCTTTCAATGTGAAACATATG -3′

EP 0 240 105 B1

# FIG. 8

B : BamHI
E : EcoRI
H : HindⅢ
M : MaeⅢ
N : NdeI
P : PstI

# FIG. 9

DERIVED FROM BACILLUS LICHENIFORMIS | SYNTHETIC DNA | DERIVED FROM BACILLUS SPHAERICUS

5' --- GTACTTGTTAAAAA TTCGGAATAT TTATACAATA TCATATGTTT CACATTGAAA GGGGAGGAGA ATC | ATG AAT TGG TTA CAA TTA GCA ---3'

-35 REGION     -10 REGION     S/D SEQUENCE | MET Asn Trp Leu Gln Leu Ala

bioB O.R.F.

HOMOLOGOUS WITH α-AMYLASE GENE PROMOTER REGION